# EUROPEAN PATENT SPECIFICATION

(11) **EP 0 362 967 B1**
(45) Date of publication and mention of the grant of the patent: **07.09.1994**
(21) Application number: 89202508.1
(22) Date of filing: 05.10.1989
(51) Int. Cl.: C07H 19/06, C07H 19/16, C07D 401/04, C07D 473/34, A61K 31/70, A61K 31/505, A61K 31/52

(54) **Nucleoside derivatives**
Nukleosid-Derivate
Dérivés de nucléosides

(30) Priority: 05.10.1988 GB 8823320
(43) Date of publication of application: 11.04.1990
(73) Proprietor: NYCOMED IMAGING AS, 0401 Oslo (NO)
(72) Inventor: Klaveness, Jo, 0276 Oslo 2 (NO); Rise, Frode, 21131 Malmö (SE); Undheim, Kjell, 1300 Sandvika (NO); Hatlelid, Jostein, 0461 Oslo 4 (NO)
(74) Representative: Holmes, Michael John

(56) References cited:
- EP-A- 0 217 580
- EP-A- 0 280 128
- EP-A- 0 311 100
- WO-A-88/07532
- WO-A-88/09332

## Description

This invention relates to antiviral compounds and more particularly to esters and amides of nucleoside derivatives which are active against human immunodeficiency virus (HIV), the retrovirus which causes the disease AIDS.

AIDS is a relatively new disease. It was discovered in 1981 and several thousand cases of the disease have been diagnosed since then. It is anticipated that the number will increase to at least several hundred thousand in the next few years. The situation is especially severe in several Central African countries. AIDS is fatal, and about 40% of all diagnosed cases have ended in death. Of those diagnosed as having AIDS three or more years ago it is estimated that 85% are now dead.

Clinical symptoms are weight loss, chronic diarrhoea, persisting fever and opportunistic infections due to loss of T-cells, thus upsetting the overall balance of the immune system. The patient loses his/her ability to combat otherwise insignificant infections.

Several different methods to combat the infection have been tried. Among the methods tried are stimulation of the immune system and conventional treatment of the (secondary) life-threatening infections. So far the most promising method has been to attack the replication of the HIV-virus. Several different compounds interfering with replication have been tried, e.g. phosphonoformate (Foscarnet), suramin, Evans Blue, 3'-azido-3'-deoxythymidine (AZT) and 2', 3'-dideoxynucleosides.

European Patent Applications Nos. 0196185A and 0217580A, for instance, describe AZT and derivatives and analogues thereof. AZT is a known compound which has shown great promise in the treatment of AIDS and AIDS-related complex. It is believed that AZT works by inhibiting reverse transcriptase, a vital enzyme in the life cycle of retroviruses.

Further work has been done on alternative reverse transcriptase inhibitors which might avoid the limitations and drawbacks of AZT, for instance bone marrow suppression or the need for frequent administration of relatively large quantities, and among those suggested have been the 2′,3′-dideoxynucleosides.

The synthesis and activity of these compounds have been described (Mitsuya and Broder, Proc. Natl. Acad. Sci. 83, 1911 (1986)) and it was demonstrated that both the 2′ and 3′ positions must be unsubstituted while the 5′-hydroxy group must be present, presumably to allow in vivo conversion to the corresponding nucleotides.

European Patent Application No 0206497A discloses 2′,3′-dideoxyribofuranoside derivatives of cytosine or purine bases as antiviral compounds. While there is reference to esters of these compounds as possible metabolic precursors, there is no suggestion that esters would possess any advantageous properties compared with the parent 5′-hydroxy compounds and no esters are specifically named or their synthesis exemplified. There is no reference to any corresponding thymidine compounds or of any nucleoside derivatives having N-acylated amino groups.

We have now found that esterification or etherification of the 4- or the 5′- oxygen and/or amidation of exocylic or endocyclic nitrogen atoms present in the purine or pyrimidine ring can give significant advantages in terms of uptake, overall activity and site of action. Our copending PCT Application PCT/GB88/00224 describes certain esters and amides of this type carrying acyl groups at the 5′ position or on exocyclic nitrogens; the present invention extends this principle to a wider range of related compounds.

Thus according to one feature of the invention we provide compounds of formula (I) 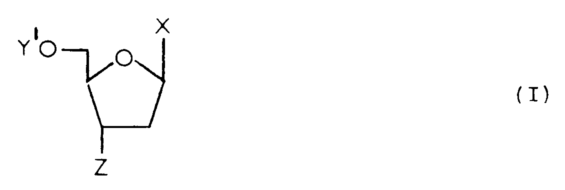
(wherein Z is a hydrogen atom or an azido group,
Y¹ is a hydrogen atom or a physiologically acceptable group of the formula

R¹(O)ₙCO(OCR²R³)ₘ-

where n is O or 1, m is 0 or 1 and
R¹ is an optionally substituted alkyl or aryl group or, where n is 0, a hydrogen atom.

R² and R³ are independently hydrogen atoms or lower alkyl groups; and
X is a group selected from 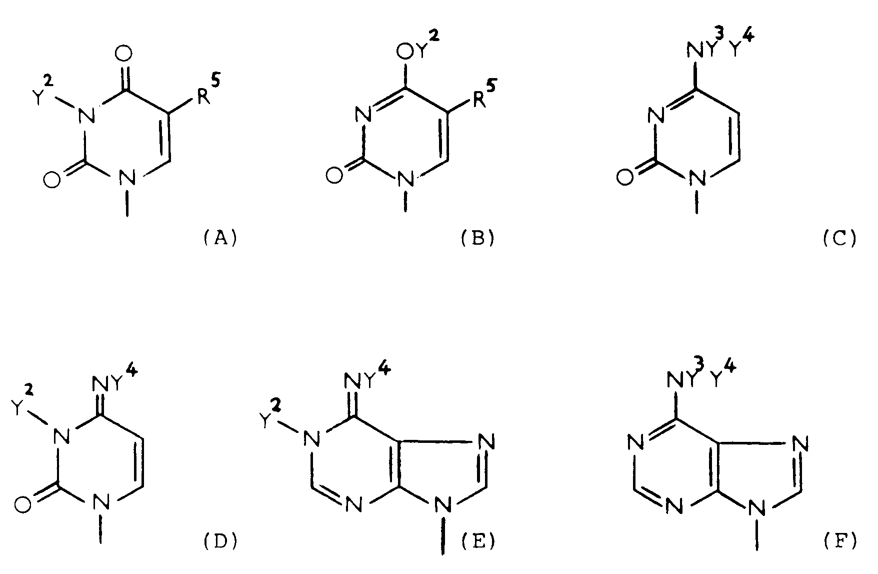 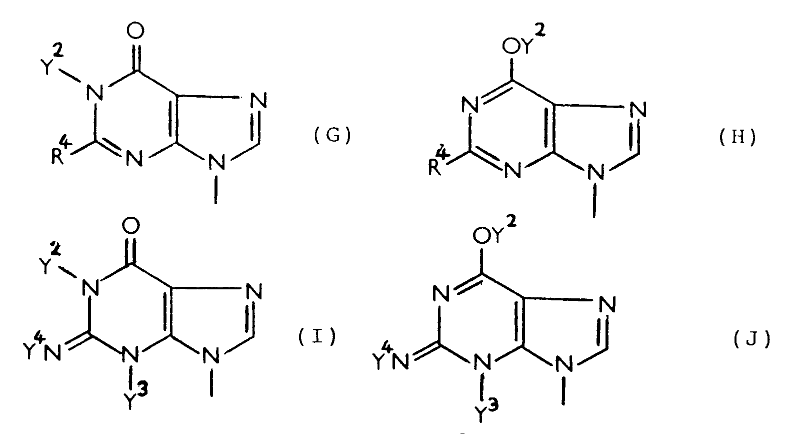
where the groups Y², Y³ and Y⁴ are as defined for Y¹ and may be the same as or different from Y¹ or each other, R⁴ is a hydrogen atom or a group -NY³Y⁴, where Y³ and Y⁴ have the above meanings and R⁵ is a hydrogen atom or a lower alkyl group, with the proviso that at least one of the groups Y¹, Y², Y³ and Y⁴ is a group R¹(O)ₙCO(OCR²R³)ₘ in which m is 1) and/or salts thereof.

It will be appreciated that some of the groups X, for example those in which Y² is a hydrogen atom, are tautomers of other of the groups X and exist in equilibrium with them

According to a further feature of the invention we provide for the use of compounds of formula (I) as hereinbefore defined, and/or salts thereof, in the manufacture of a composition for the treatment or prophylaxis of retrovirus infections, in particular neurotropic viruses and especially HIV infections. Such compositions also form part of the invention.

The group R¹ is preferably an alkyl group containing 1-20 carbon atoms which may be straight or branched, or an aryl group which may contain 6 to 20 carbon atoms and may be mono- or poly-cyclic. Substituents which may be present on the alkyl groups R¹ include aryl groups preferably having 6-10 carbon atoms (as in aralkyl groupings), hydroxy and carboxy groups. Aryl groups include heterocyclic aryl groups such as pyridinyl and thienyl groups. Substituents which may be present on aryl groups include alkyl groups, e.g. having 1-6 carbon atoms, hydroxy and carboxy groups. Examples of such groups include methyl, ethyl, propyl, t-butyl, pentyl, stearyl, palmityl, carboxyethyl and benzyl groups.

The lower alkyl groups R², R³ and R⁵ preferably contain 1-6 carbon atoms. However, R² preferably represents a hydrogen atom. R³ is preferably a hydrogen atom or more preferably a methyl group R⁵ is preferably a hydrogen atom or a methyl group.

It will be noted that the compounds of the invention may carry more than one of the groups Y¹, Y², Y³ and Y⁴. In the compounds of formula (I) D,E, I and J, it is preferred that m in the group Y⁴ is O (zero). Groups Y² are preferably of the formula

R¹.CO-, R¹CO.O.CR²R³ or R¹.O.CO.O.CR²R³-.

The salts of the compounds of formula (I) may be acid addition salts with organic or inorganic acids, for instance hydrochloric or phosphoric acid or methanesulphonic acid, ethane disulphonic acid, 2-naphthylsulphonic acid, pivalic acid and pamoic acid. Antiviral counter-ions such as phosphonoformate or suramin may also be used. Organic or inorganic base salts may be formed with acidic groups present in the molecule; suitable counter-ions include alkali metal ions such as sodium and potassium ions, divalent ions such as calcium and zinc ions and organic ions such as tetraalkylammonium and choline or ions derived from meglumine or ethylene-diamine. Salts according to the invention may be formed by reaction of the compound of formula (I) with an appropriate acid or base.

The compositions according to the invention may be used in the treatment and/or prophylaxis of retrovirus infections, in particular HIV infections. They may be formulated in conventional manner by admixture of one or more compounds of formula (I) as defined above with excipients and/or carriers.

It is believed that the compounds of formula (I) are not themselves inhibitors of reverse transcriptase but are converted in vivo to the 5-hydroxy-2′,3′-dideoxynucleosides. Nevertheless the substitution at the respective O- and N-atoms gives surprising advantages in terms of uptake and sustained activity. The compounds of formula (I) are more lipophilic than the parent compounds and this permits rapid and efficient absorption from the gastro-intestinal tract; the absorption rate may be optimised by careful choice of the substituent group to give the desired balance of lipophilicity and hydrophilicity. The lipophilic nature of the compunds of formula (I) also gives the molecules the ability to penetrate the cell membranes more easily and leads to higher intracellular concentrations, giving an improved dose/effect ratio. The steady hydrolysis of the compounds ensures a sustained concentration of the active compound in the cell and thereby permits longer intervals between doses, overcoming a significant drawback of the prior art compounds such as AZT.

Finally, the compounds according to the invention can penetrate the blood-brain barrier and thus permit treatment of the neurological disorders which have been observed to be related to the presence of neurotropic viruses, e.g. retroviruses such as HIV, and lentiviruses (Yarchoan et al, The Lancet, January 17, 1987, page 132). This is a significant advantage compared to the corresponding unsubstituted compounds or other antiviral compounds and is not referred to anywhere in the prior art, for instance in EP-A-0196185 or in EP-A-0206497. Attempts have been made to treat these neurological disorders with AZT but with limited success.

The invention thus further provides a method of treatment of neurological disorders caused by neurotropic viruses wherein an effective dose of a compound of formula (I) or a salt thereof is administered to a patient suffering from such a disorder.

Compounds of formula (I) may be prepared in any convenient way, for example, by reaction of a compound of formula (II) 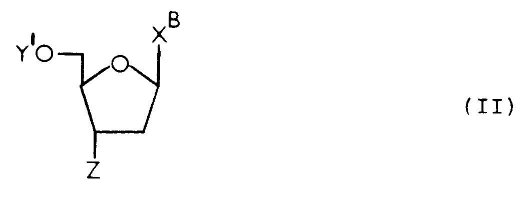
[wherein Y¹ and Z are as hereinbefore defined and X^{B} is as hereinbefore defined for X except that any of the groups Y¹, Y², Y³ and Y⁴ may each additionaly represent a protecting group, with the proviso that at least one of Y¹, Y², Y³ and Y⁴ is a hydrogen atom] with a reagent serving to introduce a group R¹(0)ₙCO.(OCR²R³)ₘ as defined above followed where required by removal of any protecting groups and/or unwanted substituents so introduced.

It should be noted that where, in the starting material, more than one of Y¹, Y², Y³ and Y⁴ is hydrogen, multiple reactions may occur.

Where it is desired to ensure that acylation or alkylation is effected while one or more groups Y¹, Y², Y³ and Y⁴ remain as hydrogen atoms, it may be desirable to protect the latter first, to form a compound of formula (I) in which one or more of Y¹, Y², Y³ and Y⁴ are protecting groups, these being removed after introduction of the desired acyl or ether group. Such protecting groups may, in fact, be conventional N- or O-protecting groups including groups R¹OCO- which may be selectively removed in the presence of the group(s) intended to remain. Thus, for example, an N-benzyloxycarbonyl may be used to protect an exocylic amino group and if the group which is intended to remain is not one which is removable by reduction, for example a straight chain alkoxycarbonyl group, the N-benzyloxycarbonyl group can readily be removed selectively using hydrogen and a noble metal catalyst such as palladium. Trisubstituted silyl groups may also be used as protecting groups, especially for the 5′-oxygen atom, and include trialkylsilyl e.g. trimethylsilyl, dimethyl-t-butylsilyl, and thexyldimethyl silyl groups.

In general, where more than one of Y¹, Y², Y³ and Y⁴ are hydrogen, and a mixture or compounds is produced, the individual components may readily be separated, for example by chromatography.

Where 5′-0-monoalkylation is to be effected (i.e. introduction of a group Y¹ in which m is 1) it is especially effective to form a dianion of the nucleoside (e.g. by reacting with sodium hydride) and to react this with one equivalent of the alkylating agent. It is of course, still possible to use protected forms of the nucleoside, for example by acylation of a nucleophilic nitrogen atom before salt formation with sodium hydride.

Suitable acylating agents for use in the reaction have the formula Ac-L where L is a leaving group. When the acyl group Ac- is derived from a carboxylic acid, i.e. is of formula R¹-CO-, then suitable acylating agents include the acid halides and acid anhydrides advantageously in the presence of a base; when the acyl group is derived from a carbonic acid, i.e. is of formula R¹.O.CO-, then acylating agents include the haloformate esters and reactive carbonic acid diesters. In such reagents, the halogen may, for example, be chlorine or bromine. The base for use in the reaction with the acid halide or anhydride may, for example, be a heterocyclic base such as pyridine or 4-dimethylaminopyridine. The latter increases the speed of the reaction and may be used advantageously with pyridine. The reaction will normally be carried out in the presence of an inert solvent e.g. a substituted amide solvent such as dimethylformamide, dimethylacetamide or a halogenated hydrocarbon such as dichloromethane.

In general, we have found that using acid anhydrides as acylating agents to introduce a group R¹CO, O-acylation at the 5′-position takes place more readily than N-acylation, whereas using acid halides, N-acylation or even N-diacylation predominates. However, N-acyl groups R¹CO- may be removed selectively, for example by reaction with a phenol such as p-methyl-phenol. Where multiple substitution is to be effected, a stronger base such as sodium hydride may be advantageous.

Suitable acyloxyalkylating agents for use in the invention will in general be of the formula R¹CO.O.CR²R³L or R¹O.CO.O.CR²R³L, where L is a leaving group. Thus, the group L may for example, be a halogen atom such as a chlorine or bromine atom or a hydrocarbon-sulphonyloxy group such as a tosyloxy or mesyloxy group.

The alkylation reaction will normally be effected in the presence of a base, conveniently an inorganic carbonate such as potassium carbonate or an alkali metal hydride such as sodium hydride. Bases as used for acylation may also be useful.

The starting compounds of formula (II) wherein Y¹, Y², Y³ and Y⁴ are all hydrogen atoms are well described in the literature - see, for instance, Lin et al, J. Med. Chem. 30, 440 (1987). Starting compounds wherein one or more of Y¹, Y², Y³ and Y⁴ are other than hydrogen may be prepared by preliminary reactions as described above.

The pharmaceutical compositions according to the invention may be formulated conventionally by means well known in the art, and may be administered by any convenient route, for instance orally, rectally, vaginally, intraveneously or intramuscularly. Examples of suitable formations include tablets and capsules, aqueous formulations for intravenous injection and oil-based formulations for intramuscular injection. Suitable dosages will lie in the range 0.1 to 100mg per kilogram of bodyweight per 24 hour period. The compositions according to the invention may also contain other active antivirals for instance acyclovir, phosphonoformate, suramin, Evans Blue, interferons or AZT.

The invention is illustrated by the following Examples (starting materials are either known materials or are prepared according to our copending PCT Application PCT/GB88/00224). Capsugel is a Trade Mark.

### Example 1

### 3-Acetoxymethyl-2′,3′-dideoxyuridine (Formula (I)A, Y² = acetoxymethyl, R⁵=Y¹=H)

A mixture of 2′,3′-dideoxyuridine (1 mmol) and potassium carbonate (0.8 mmol) in dry N,N-dimethylacetamide (20 ml) is stirred at ambient temperature for 2 hours, a solution of acetoxymethyl chloride (0.7 mmol) in dry N,N-dimethylacetamide (5 ml) added dropwise with stirring, the mixture stirred at ambient temperature for 36 hours, filtered, the filtrate evaporated and the product purified by flash chromatography on silica gel using ethyl acetate:light petroleum.

### Example 2

### 3,5′-O-Bis(acetoxymethyl)-2′,3′-dideoxyuridine (Formula (I)A, Y¹=Y² = acetoxymethyl, R⁵=H) and O⁴, 5′-O-bis(acetoxymethyl-2′,3′-dideoxyuridine (Formula (I)B,Y¹=Y² = acetoxymethyl, R⁵=H)

A mixture of 2′,3′-dideoxyuridine (1 mmol) and sodium hydride (2 mmol) in dry DMF (50 ml) is stirred at ambient temperature for 2 hours, a solution of acetoxymethyl chloride (2 mmol) in dry DMF (10 ml) added dropwise with stirring, the mixture stirred at ambient temperature for 24 hours, filtered, the filtrate evaporated and the product mixture separated by flash chromatography on silica gel using ethyl acetate:light petroleum.

### Example 3

### 3-Acetoxymethyl-5′O-palmitoyl-2′,3′-dideoxythymidine (Formula (I)A, Y² = acetoxymethyl, Y¹=palmitoyl, R⁵=methyl)

A mixture of 5′-O-palmitoyl-2′,3′-dideoxythymidine (1 mmol) and potassium carbonate (0.8 mmol) in dry N,N-dimethylacetamide (20 ml) is stirred at ambient temperature for 2 hours, a solution of acetoxymethyl chloride (0.7 mmol) in dry N,N-dimethylacetamide (5 ml) added dropwise with stirring, the mixture stirred at ambient temperature for 24 hours, filtered, the filtrate evaporated and the product purified by flash chromatography on silica gel using ethyl acetate:light petroleum.

### Example 4

### N⁴,5′-O-Di(benzyloxycarbonyl)-N⁴-pivaloyloxymethyl-2′,3′-dideoxycytidine (Formula (I)C, Y¹=Y⁴ benzyloxycarbonyl, Y³ = pivaloyloxymethyl) and 3-pivaloyloxymethyl-N⁴,5′-0-di(benzyloxycarbonyl) 4-imino-3,4-dihydro-2′,3′-dideoxycytidine (Formula (I) D, Y²=pivaloyloxymethyl, Y¹= Y⁴ = benzyloxycarbonyl)

N⁴,5′-O-Di(benzyloxycarbonyl)-2′,3′-dideoxycytidine (1 mmol) is dissolved in dry DMF (20 ml), the solution cooled to 0°C, sodium hydride (1.1 mmol) added, the mixture stirred at 0°C for 1 hour when the bubbling of hydrogen has ceased, pivaloyloxymethyl chloride (1.1 mmol) in dry DMF (10 ml) added dropwise, the resultant mixture stirred at ambient temperature for 36 hours, most of the solvent removed below 40°C using an oil pump, water added to the residue and the mixture extracted with ethyl acetate. The dried (MgSO₄) ethyl acetate solution is evaporated and the residue subjected to flash chromatography using light petroleum: ethyl acetate which elutes the title compound.

### Example 5

### N⁴-Pivaloyloxymethyl-2′,3′-dideoxycytidine (Formula (I)C, Y¹=Y⁴=H, Y³=pivaloyloxymethyl)

N⁴,5′-O-Di(benzyloxycarbonyl)-N⁴-pivaloyloxymethyl-2′,3′-dideoxycytidine (0.2 mmol) is added to a suspension of 5% palladium on charcoal (25 mg) in ethanol (25 ml), the air removed under vacuum and the mixture hydrogenated at atmospheric pressure until the hydrogenolysis is completed (ca. 3 hours). The mixture is then filtered, the filtrate evaporated at reduced pressure and the product purified by filtration (chromatography) on neutral silica gel using chloroform:ethanol.

### Example 6

### 3-Pivaloyloxymethyl-3′-deoxythymidine

i) 3′-Deoxythymidine (0.0449 g, 0.198 mmol) and imidazol (0.0328 g, 0.482 mmol) were dissolved in DMF (0.5 ml). Thexyldimethylsilyl chloride (0.047 ml, 0.238 mmol) was added and the reaction mixture was stirred at room temperature for 19 hours. The solvent was removed under reduced pressure and chloroform (15 ml) was added to the residue, washed with water (5ml x 2), dried (MgSO₄) and evaporated. The residue was chromatographed on silica using ethyl acetate-hexane (7:3); yield 0.0578 g (79%) of 5′-0-thexyldimethyl-silyl-3′-deoxythymidine as a white powder. Mp. 109-110°C (uncorrected).
   ¹H NMR (CDCl₃, 300 MHz) δ :0.14(s, 6H),0.86-0.90 (m, 12H),1.64(m,1H),1.91(s,3H), 1.93-2.02 (m,3H)2.26-2.41(m,1H),3.66-3.96(ABX,2H),4.08-4.17(m,1H),6.05(dd,1H),7.51 (d,1H),8.50(b,1H).
   ¹³C NMR(CDCl₃)δ :-3.51, -3.43, 12.47, 18.31, 18.41, 20.12, 20.32, 25.32, 25.38, 32.30, 33.91, 64.24, 80.76, 85.68, 110.18, 135.53, 150.14, 163.59.
ii) The 5′-0-thexyldimethylsilyl-3′-deoxythymidine (0.0254 g, 0.0689 mmol) and potassium carbonate (0.0111 g, 0.0803 mmol) were suspended in DMF (1ml) and stirred for 1.5 hours at room temperature. The mixture was cooled to 0°C and chloromethyl pivalate (0.013 ml, 0.0895 mmol) was added. The mixture was stirred at room temperature for 18 hours before the solvent was evaporated under reduced pressure. The residue was chromatographed on silica using ethyl acetate - hexane (35:65); yield 0.031 g (93%) of 3-pivaloyloxymethyl-5′-0-thexyldimethylsilyl-3′-deoxythymidine as a glassy material.
   ¹H NMR(CDCl₃, 300 MHz) δ : 0.14(s,6H),0.87-0.90(m,12H), 1.17(s,9H),1.56-1.70(m,1H),1.94(d,3H),1.95-2.02(m,3H),2.30-2.44(m,1H),3.67-3.97(ABX,2H),4.11-4.17(m,1H),5.90-5.98(AB,2H),6.06(dd,1H),7.54(d,1H).
   ¹³C NMR(CDCl₃) δ: -3.49, -3.42, 13.09, 18.31, 20.12, 20.33, 25.34, 26.92, 32.37,33.91, 38.71, 64.22, 64.90, 80.92, 86.35, 109.37, 134.50, 150.23, 162.58, 177.43.
iii) The 3-pivaloyloxylmethyl-5′-0-thexyldimethylsilyl-3′-deoxythymidine (0.029 g, 0.061 mmol) was dissolved in THF (0.5 ml) and a 0.25 M solution of tetrabutylammonium fluoride in THF (0.5 ml) was added. The mixture was stirred for 30 minutes before the solvent was evaporated. The residue was dissolved in chloroform (7 ml) and washed with water (1 ml). The organic phase was dried (MgSO₄), evaporated and the residue purified by preparative TLC. The plates were eluted with diethyl ether (3x) and the product extracted from the main band by chloroform-methanol (9:1); yield 0.0170 g (83%) of the title compound as a white solid. Mp. 64-66°C (uncorrected)
   ¹H NMR(CDCl₃, 300MHz) δ: 1.17(s,9H),1.93(d,3H),1.95-2.15(m,4H),2.32-2.48(m,1H),3.72-4.03 (ABX,2H),4.14-4.21(m,1H),5.90-5.97(AB,2H),6.10(dd,1H),7.60(d,1H).
   ¹³C NMR(CDCl₃) δ: 13.12, 24.91, 26.90, 32.22, 38.71, 63.42, 64.89, 81.10, 86.73, 109.62, 135.04, 150.26, 162.51, 177.47.

### Example 7

### 3,5′-0-Di(pivaloyloxymethyl)-3′-deoxythymidine

3′-Deoxythymidine (0.050 g, 0.221 mmol) was dissolved in DMF (1 ml) and cooled to 0°C. Sodium hydride (0.0147 g, 80% in oil, 0.49 mmol) was added and the mixture stirred at 0°C for 1 hour before chloromethyl pivalate was added. The mixture was stirred at 0°C for 1 hour and then at room temperature for 20 hours. TLC indicated partial conversion to the dialkylated product and the mixture was cooled to 0°C and sodium hydride (0.0027 g, 80% in oil, 0.090 mmol) was added. The mixure was stirred at 0°C for 1 hour before chloromethyl pivalate (0.013 ml, 0.090 mmol) was added. The mixture was stirred for 1 hour at 0°C and at room temperature for 19 hours before the solvent was evaporated under reduced pressure. Chloroform (10 ml) was added to the residue and the mixture washed with saturated sodium chloride (5 ml x 2) and water (5 ml x 2). The chloroform phase was dried (MgSO₄), evaporated and the residue subjected to preparative TLC. The plates were eluted with diethyl ether - pentane - methanol (50:50:0.1) (7x) and the product extracted from the main band by chloroform - methanol; yield 0.042 g (42%) as a colourless oil.
¹H NMR (CDCl₃, 300 MHz) δ: 1.19(s, 9H),1.24(s,9H),1.95-2.10(m,6H),2.30-2.50(m,1H),3.74-4.06(ABX,2H),4.20-4.30(m,1H),5.33-5.40(AB,2H),5.92-5.99(AB,2H),6.10-6.16(M,1H),7.66(d,1H).
¹³C NMR (CDCl₃) δ: 13.04, 25.13, 26.91, 32.51, 38.70, 38.84, 64.87, 70.16, 79.42, 86.40, 88.49, 109.44, 134.83, 150.24, 162.53, 177.39, 177.75.

### Example 8

### 5′-0-Pivaloyloxymethyl-3′-deoxythymidine

A mixture of 3′-deoxythymidine (0.0103 g, 0.046 mmol) and sodium hydride (0.0029 g, 80% in oil, 0.97 mmol) in DMF (0.5ml) was stirred at 0°C for 1 hour. Chloromethyl pivalate (0.0070 ml, 0.048 mmol) was added and the mixture stirred at 0°C for 3 hours. Ammonium chloride 1M (3 ml) was added and the mixture extracted with diethyl ether (3 ml). The ether extract was washed with saturated sodium chloride (3 ml x 2), dried (MgSO₄) and evaporated. The residue was chromotographed on silica using chloroform - methanol; yield 0.0068 g (43%) as a glassy material.
¹H NMR (CDCl₃, 300 MHz) δ: 1.23(s,9H),1.90-2.18(m,6H), 2.30-2.50(m,1H),3.73-4.05(ABX,2H),4.18-4.28(m,1H),5.33-5.39(AB,2H),6.08-6.14(m,1H),7.62(d,1H),8.44(b,1H).
¹³C NMR (CDCl₃) δ: 12.59, 25.29, 27.03, 32.58, 38.97, 70.33, 79.40, 85.83, 88.60, 110.37, 136.01, 150.26, 163.69, 177.92.

### Example 9

### 5′-0-Pivaloyloxymethyl-3′-azido-3′-deoxythymidine

A mixture of 3′-azido-3′-deoxythymidine (0.0335 g, 0.125 mmol) and sodium hydride (0.0079 g, 80% in oil, 0.263 mmol) in DMF (1.5 ml) was stirred at 0°C for 1.5 hours. Chloromethyl pivalate (0.020 ml, 0.138 mmol) was added and the mixture stirred at 0°C for 1 hour. Acetic acid (0.0072 ml, 0.126 mmol) was added and the solvent evaporated. The residue was chromatographed on silica using chloroform - methanol; yield 0.031 g (63%) as an oil.
¹H NMR(CDCl₃, 300 MHz) δ: 1.22(s,9H),1.94(d,3H),2.20-2.50(m,2H),3.76-4.02(m,3H)4.22-4.32(m,1H),5.34(s,2H), 6.23(t,1H),7.44(d,1H),9.35(b,1H).
¹³C NMR(CDCl₃) δ: 12.51, 26.91, 37.71, 38.90, 59.91, 68.49, 82.58, 84.55, 88.27, 111.32, 135.39, 150.38, 163.88, 177.88.

### Example 10

### N⁴-Benzyloxycarbonyl-5′-0-pivaloyloxymethyl-2′,3′-dideoxycytosine

A mixture of N⁴-Benzyloxycarbonyl-2′,3′-dideoxycytosine (0.022 g, 0.0637 mmol) and sodium hydride (0.0044 g 80% in oil, 0.147 mmol) in DMF (1.5 ml) was stirred at 0°C for 1.5 hours. The mixture was cooled to 50°C and chloromethyl pivalate (0.0102 ml, 0.0702 mmol) was added. The mixture was stirred at -50°C for 3.5 hours. Saturated ammonium chloride (1 ml) was added and the solvents evaporated. The residue was chromatographed on silica using chloroform-methanol; yield: 0.0145 g (50%) as an oil.
¹H NMR(CDCl₃, 300 MHz) δ: 1.24(s,9H),1.80-2.00(m,2H),2.10-2.25(m,1H),2.42-2.60(m,1H),3.73-4.12(ABX,2H),4.22-4.34(m,1H),5.23(s,2H),5.27-5.43(AB,2H),6.07(dd,1H),7.15-7.30(b,1H),7.30-7.48(m,6H),8.32(d,1H).

### Example 11

### 5′-0-Pivaloyloxymethyl-2′,3′-dideoxycytosine

N⁴-Benzyloxycarbonyl-5′-0-pivaloyloxymethyl-2′,3′-dideoxycytosine (0.05 mmol) is added to a suspension of 5% Pd on charcoal (5 mg) in ethanol (2 ml). The stirred suspension is subjected to hydrogenolysis at atmospheric pressure using a Brown apparatus where the H₂- gas is generated in a controlled manner by the addition of 3N HCl to a solution of sodium borohydride in a separate compartment. The reaction is run for 1 hour at room temperature, the mixture filtered through a thin bed of Celite, the filtrate evaporated and the product purified by chromatography on a silica gel column using CHCl₃:EtOH(9:1) for elution.

### Example 12

### 3-Pivaloyloxymethyl-5′-0-propionyl-3′-azido-3′-doexythymidine

3′-azido-3′-deoxythymidine (0.0504g, 0.189 mmol) and 4-dimethylaminopyridine (0.0023g, 0.019 mmol) were dissolved in pyridine (2ml) and cooled to 0°C. Propionic anhydride (0.0265 ml, 0.206 mmol) was added and the mixture stirred at room temperature under nitrogen for 24 hours before the solvent was evaporated under reduced pressure. Toluene was added and evaporated under reduced pressure and the residue chromatographed on silica using chloroform and chloroform-methanol (98.2); yield 0.060 g, (98%) of 5′-O-propionyl-3′-azido-3′-deoxythymidine as a colourless oil.

5′-O-propionyl-3′-azido-3′-deoxythymidine (0.042 g, 0.130 mmol) and potassium carbonate (0.0198g, 0.143 mmol) were suspended in DMF (1ml) and stirred at room temperature under nitrogen for 1.5 hours. The mixture was cooled to °C and chloromethyl pivalate (0.0226 ml, 0.156 mmol) added. The mixture was stirred at 0°C for 30 minutes and then at room temperature for 19 hours before the solvent was evaporated under reduced pressure. The residue was chromatographed on silica using ethyl acetate-hexane (4:6); yield 0.045g (81%) as a colourless oil.
¹H NMR (CDCl₃, 300 MHz) δ: 1.19 (s) and 1.19(t) [12H], 1.96 (d,3H), 2.29-2.57 (m) and 2.41 (q) [4H], 4.06-4.15 (m,1H), 4.16-4.25 (m,1H), 4.31-4.42 (ABX, 2H), 5.91-5.98 (AB,2H), 6.15 (t,1H), 7.27 (d,H-6).
¹³C NMR (CDCl₃) δ: 9.03, 13.27, 27.02, 27.43, 37.73, 38.84, 60.54, 63.19, 64.95, 81.91, 86.10, 110.49, 134.10, 150.04, 162.27, 173.72, 177.48.

### Example 13

### 3-α-(Ethyloxycarbonyloxy)ethyl-5′-O-propionyl-3′-deoxythymidine

3′-Deoxythymidine (0.0352g, 0.156 mmol) and 4-dimethylaminopyridine (0.0020 g, 0.016 mmol) were dissolved in pyridine (3ml) and cooled to 0°C. Propionic anhydride (0.0241 ml, 0.187 mmol) was added and the mixture stirred at room temperature under nitrogen for 24 hours before the solvent was evaporated under reduced pressure. Toluene was added and evaporated under reduced pressure and the residue chromatographed on silica using chloroform and chloroform-methanol (98,2); yield 0.0435 g (99%) of 5′-O-propionyl-3′-deoxythymidine as a semi-solid.

5′-0-propionyl-3′-deoxythymidine (0.063 g, 0.223 mmol) and potassium carbonate (0.0339 g, 0.245 mmol) were suspended in DMF (2ml) and stirred at room temperature under nitrogen for 1.5 hours. The mixture was cooled to 0°C and 1-chloroethylethylcarbonate (0.039 ml, 0.291 mmol) was added. The mixture was stirred at 0°C for 30 minutes and at room temperature for 2 hours. The temperature was increased to 60°C and the mixture stirred for 19.5 hours before the solvent was evaporated under reduced pressure. The residue was chromatographed on silica using ethyl acetate-hexane (50:50) (R_{f}0.17); yield 0.0477 g (54%) as an oil.
¹H NMR (CDCl₃, 300 MHz) δ: 1.18 (t,3H), 1.29 (t,3H), 1.70-1.87 (m) and 1.86 (d) [4H], 1.94 (d,3H), 1.97-2.14 (m,2H), 2.35-2.52 (m) and 2.40 (q) [3H], 4.12-4.25 (m,2H), 4.26-4.40 (m,3H), 6.07-6.12 (m,1H), 7.20-7.28 (m,1H), 7.36 (d, 1H).
¹³C NMR (CDCl₃) δ:9.09, 13.23 and 13.36* 14.15, 17.86 and 17.94* 25.81 and 25.84* 27.48, 32.25 and 32.35* 64.34, 64.79, 77.49, 78.46 and 78.56* 86.52 and 86.58* 109.81 and 109.84* 133.92 and 133.97* 149.82, 153.81 and 153.85* 162.57 and 162.59* 174.06.
* Different shifts for the two enantiomers (due to )-CH(CH₃)-N) are observed.

### Example 14

### 5′-O-Propionyl-3-α-(thienyloxycarbonyloxy)ethyl-3′-azido-3′-deoxythymidine

5′-O-Propionyl-3′-azido-3′-deoxythymidine (0.051 g, 0.158 mmol) and potassium carbonate (0.024 g, 0.174 mmol) were suspended in DMF and stirred at room temperature under nitrogen for 1 hour. The mixture was cooled to 0°C and 1-chloroethyl thienyl carbonate (0.046 g, 0.208 mmol) added. The mixture was stirred at room temperature for 17 hours and then at 55°C for 9 hours. The solvent was evaporated under reduced pressure and the residue chromatographed on silica using chloroform and chloroform - methanol (99:1). The product containing fractions, R_{f} 0.3 (chloroform - methanol (99:1) were composed of two diastereoisomeric components, R_{f}0.18 and R_{f}0.22 (ethyl acetate - hexane (4:6); yield 0.050 g.

### Example 15

### N⁶-Benzyloxycarbonyl-5′-O-pivaloyloxymethyl-2′,3′-dideoxyadenosine

2′,3′-Dideoxyadenosine (0.1 mmol) and 4-dimethylaminopyridine (0.1 mmol) were dissolved in pyridine and the mixture cooled to 0°C. Benzyl chloroformate (0.2 mmol) was added and the resulting mixture stirred at room temperature under nitrogen for 24 hours. 4-Dimethylaminopyridine (0.1 mmol) was added and the mixture cooled to 0°C. Benzyl chloroformate (0.2 mmol) was added and the mixture stirred at room temperature for 24 hours. The addition of 4-dimethylaminopyridine and benzyl chloroformate was repeated 3 times with 24 hours of stirring between each addition before the solvent was evaporated under reduced pressure. The residue was chromatographed on silica using chloroform, chloroform-methanol (99:1) and chloroform-methanol (9:1) yielding N⁶-benzyloxycarbonyl-2′,3′-dideoxyadenosine. N⁶-benzyloxycarbonyl-2′,3′-dideoxyadenosine (0.1 mmol) and sodium hydride (80% in oil, 0.21 mmol) in DMF (2 ml) was stirred at 0°C for 1 hour. The mixture was cooled to -50°C and chloromethylpivalate (0.1 mmol) added. The mixture was stirred for 4 hours before acetic acid (0.1 mmol) was added. The solvent was evaporated under reduced pressure and the residue chromatographed on silica using chloroform and chloroform-methanol (99:1), yielding the title compound.

### Pharmaceutical Example A

### Preparation of capsules for oral use

| | |
|---|---|
| Active Compound | 50 mg |
| Amylum maydis | q.s. |

The powder is mixed and filled into hard gelatin capsules (Capsugel Size 00).

### Pharamceutical Example B

### Preparation of an ointment

| | |
|---|---|
| Active compound | 1 g |
| Liquid paraffin | 100 g |
| White soft paraffin | to 1000 g |

White soft paraffin is melted and incorporated into the liquid paraffin and stirred until the mixture is cold. Active compound is triturated with a portion of the basis and gradually the remainder of the basis was incorporated. The ointment is filled into lacquered aluminium tubes (20 g) and sealed. The ointment contains 0.1 % active compound.

### Pharmaceutical Example C

### Suspension for parenteral administration

| | |
|---|---|
| Active Compound | 200 gram |
| Polysorbate 80 | 3 gram |
| Sorbitol | 400 gram |
| Benzyl alcohol | 8 gram |
| Water | ad 1000 ml |
| 1M HCl | q.s. |

Polysorbate 80, Sorbitol and benzyl alcohol are dissolved in 500 ml distilled water. Active compound is screened through a 0.15 mm sieve and dispersed in the solution under vigorous stirring. The pH is adjusted to 4.5 by dropwise addition of 1M HCl. Water is added to 1000 ml, the suspension was filled in 1 ml vials The vials are sterilized by γ-radiation. Each vial contains 200 mg active compound.

### Pharmaceutical Example D

### Preparation of tablets

| | Gram |
|---|---|
| Active Compound | 200 |
| Lactose | 85 |
| Polyvinylpyrrolidone | 5 |
| Starch | 42 |
| Talcum powder | 15 |
| Magnesium stearate | 3 |

Active compound and lactose are screened through a 0.15 mm sieve and mixed together for 10 minutes. The mixed powder is wetted with an aqueous solution of polyvinyl-pyrrolidone. The mass is granulated, and the dried (40 °C) granulate is mixed with starch, talcum powder and magnesium stearate. The granulate is compressed into tablets. The tablet diameter is 11 mm, the tablet weight is 350 mg and each tablet contains 200 mg active compound.

### Pharmaceutical Example E

### Preparation of a suspension for rectal administration

Methyl p-hydroxybenzoate (70 mg) and propyl p-hydroxybenzoate (15 mg) are dissolved in water (100 ml) at 90 °C. After cooling to 30 °C methyl cellulose (2g) is added and the mixture is agitated for 3 hours. 1 gram active compound are screened through a 0.15 mm sieve, and dispersed in the solution under vigorous stirring. The suspension is filled in a 100 ml tube. The suspension contain 10 mg active compound/ml.

### Pharmaceutical Example F

### Preparation of oral suspension

| | Gram |
|---|---|
| Active Compound | 10 |
| Carboxymethyl cellulose | 1.5 |
| Sorbitol | 200 |
| Sodium benzoate | 1.0 |
| Orange essence | 0.3 |
| Apricot essence | 0.7 |
| Ethanol | 50 |
| Water | 236.5 |

Carboxymethyl cellulose, sorbitol and sodium benzoate are dissolved in water with stirring for 2 hours. A solution of the essences in ethanol is added. Active compound is screened through a 0.15 mm sieve and dispersed in the solution under vigorous stirring. The suspension (10 gram) is filled in a 20 ml tube. Each tube contains 200 mg active compound.

### Pharmaceutical Example G

### Preparation of injection solution

10 mg active compound are dissolved in 10 ml 0.9 % sodium chloride. pH is adjusted to 4.5 with 1N HCl. The solution is sterile filtered and filled into a 10 ml vial. The solution contains 1 mg active compound/ml.

### Pharmaceutical Example H

### Preparation of tablets (controlled release formulation)

| | Gram |
|---|---|
| Active Compound | 500 |
| Hydroxypropylmethylcellulose (Methocel K15) | 120 |
| Lactose | 45 |
| Povidone | 30 |
| Magnesium stearate | 5 |

Active compound hydroxypropyl-methylcellulose and lactose are mixed together for 20 minutes and granulated with a solution of povidone. Magnesium stearate is added and the mixture is compressed into tablets. The tablet diameter is 13 mm, the tablet weight is 700 mg and each tablet contains 500 mg active compound.

## Claims (Claims for the following Contracting State(s): AT, BE, CH, DE, FR, GB, IT, LI, LU, NL, SE)

1. Compounds of formula (I) 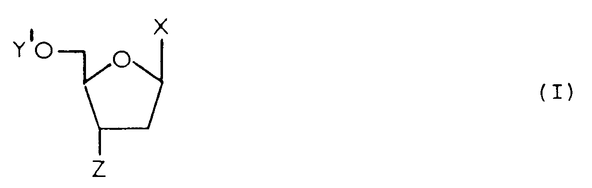 (wherein Z is a hydrogen atom or an azido group,
Y¹ is a hydrogen atom or a physiologically acceptable group of the formula
R¹(O)ₙCO(OCR²R³⁾ₘ-
where n is O or 1, m is 0 or 1 and
R¹ is an optionally substituted alkyl or aryl group or, where n is 0, a hydrogen atom.
R² and R³ are independently hydrogen atoms or lower alkyl groups; and
X is a group selected from 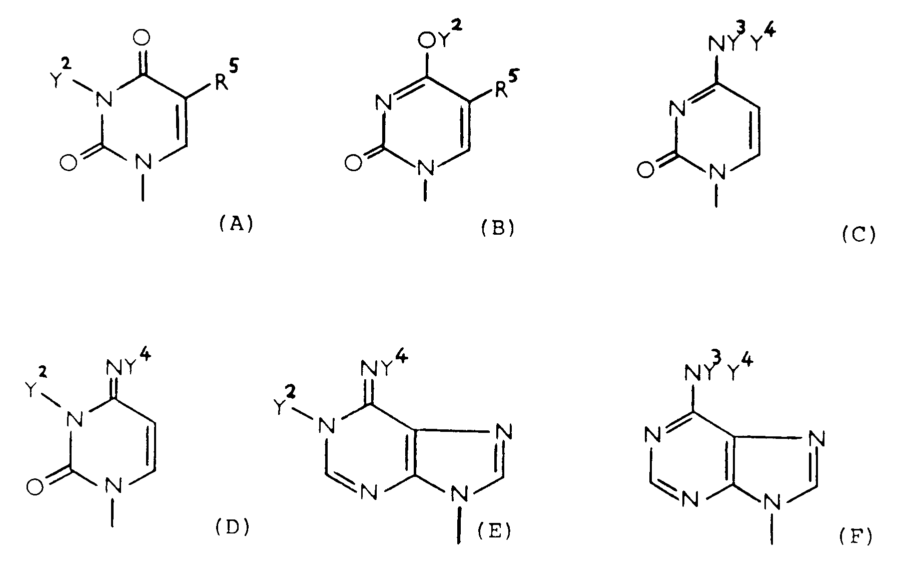 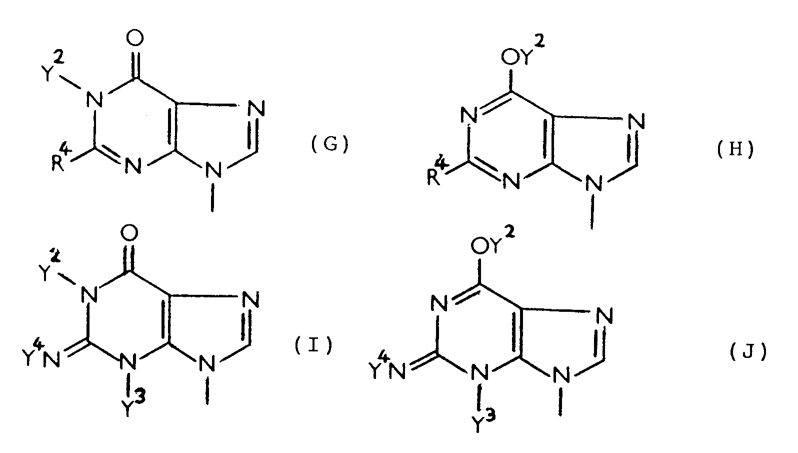 where the groups Y², Y³ and Y⁴ are as defined for Y¹ and may be the same as or different from Y¹ or each other, R⁴ is a hydrogen atom or a group -NY³Y⁴, where Y³ and Y⁴ have the above meanings and R⁵ is a hydrogen atom or a lower alkyl group, with the proviso that at least one of the groups Y¹, Y², Y³ and Y⁴ is a group R¹(O)ₙCO(OCR²R³)ₘ in which m is 1) and/or salts thereof.

2. Compounds of formula (I) as claimed in claim 1 wherein R¹ is selected from optionally substituted C₁₋₂₀ alkyl groups and C₆₋₂₀ aryl groups.

3. Compounds of formula (I) as claimed in claim 1 wherein R² is a hydrogen atom; and R³ is a hydrogen atom or a methyl group.

4. A pharmaceutical composition comprising as active ingredient one or more compounds of formula (I) as defined in any preceding claim and/or a non-toxic salt thereof, together with a pharmaceutical carrier or excipient.

5. A process for the preparation of a compound of formula (I) as defined in any of claims 1 to 3, which comprises reaction of a compound of formula (II) 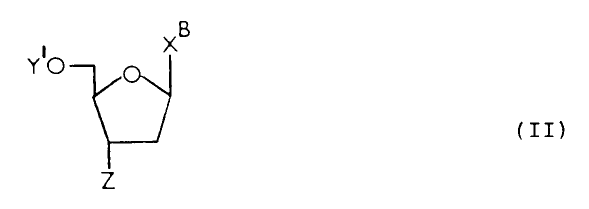 [wherein Y¹ and Z are as hereinbefore defined and X^{B} is as hereinbefore defined for X except that any of the groups Y¹, Y², Y³ and Y⁴ may each additionaly represent a protecting group, with the proviso that at least one of Y¹, Y², Y³ and Y⁴ is a hydrogen atom] with a reagent serving to introduce a group R¹(0)ₙCO.(OCR²R³)ₘ as defined above followed where required by removal of any protecting groups and/or unwanted substituents so introduced.

6. Use of compounds of formula (I) as defined in any of claims 1 to 3, and/or salts thereof, in the manufacture of a medicament for the treatment or prophylaxis retrovirus infections.

## Claims (Claims for the following Contracting State(s): ES, GR)

1. A process for the preparation of compounds of formula (I) 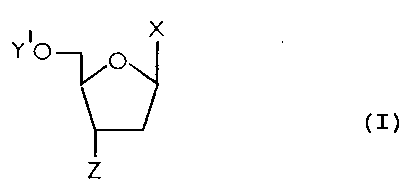 wherein Z is a hydrogen atom or an azido group,
Y¹ is a hydrogen atom or a physiologically acceptable group of the formula
R¹(O)ₙCO(OCR²R³)ₘ-
where n is O or 1, m is 0 or 1 and
R¹ is an optionally substituted alkyl or aryl group or, where n is 0, a hydrogen atom.
R² and R³ are independently hydrogen atoms or lower alkyl groups; and
X is a group selected from 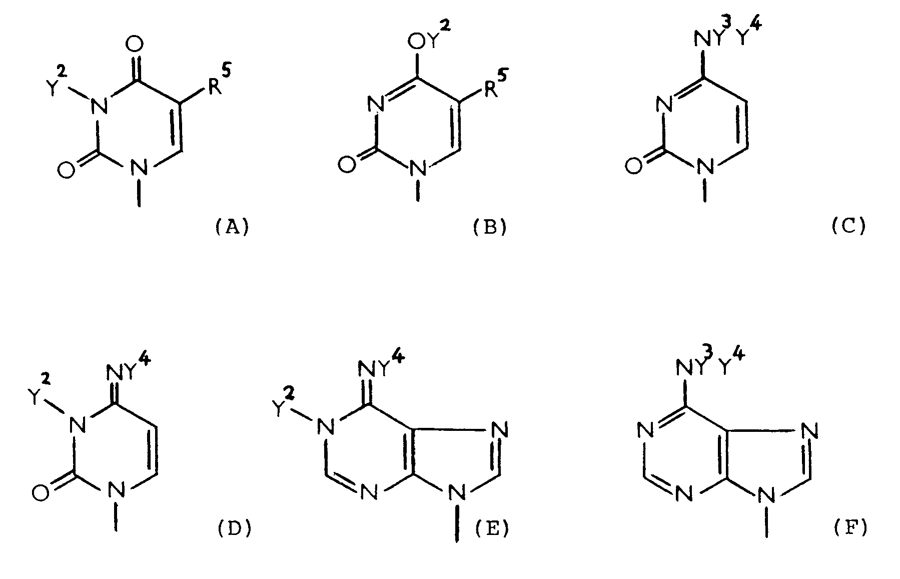 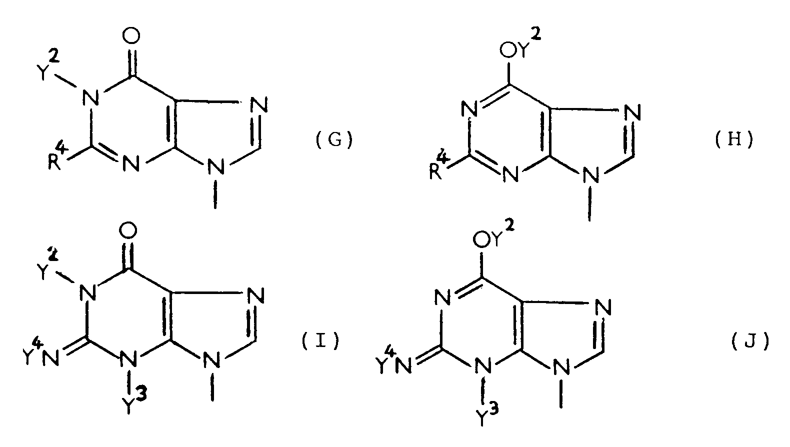 (where the groups Y², Y³ and Y⁴ are as defined for Y¹ and may be the same as or different from Y¹ or each other, R⁴ is a hydrogen atom or a group -NY³Y⁴, where Y³ and Y⁴ have the above meanings and R⁵ is a hydrogen atom or a lower alkyl group, with the proviso that at least one of the groups Y¹, Y², Y³ and Y⁴ is a group R¹(O)ₙCO(OCR²R³)ₘ in which m is 1) and/or salts thereof, comprising reaction of a compound of formula (II) 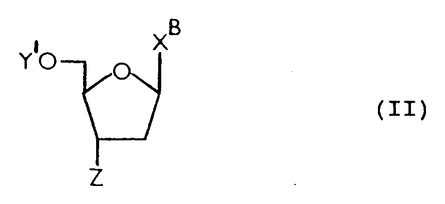 [wherein Y¹ and Z are as hereinbefore defined and X^{B} is as hereinbefore defined for X except that any of the groups Y¹, Y², Y³ and Y⁴ may each additionaly represent a protecting group, with the proviso that at least one of Y¹, Y², Y³ and Y⁴ is a hydrogen atom] with a reagent serving to introduce a group R¹(0)ₙCO.(OCR²R³)ₘ as defined above followed where required by removal of any protecting groups and/or unwanted substituents so introduced.

2. A process as claimed in claim 1 for the preparation of compounds of formula (I) wherein R¹ is selected from optionally substituted C₁₋₂₀ alkyl groups and C₆₋₂₀ aryl groups.

3. A process as claimed in claim 1 for the preparation of compounds of formula (I) wherein R² is a hydrogen atom; and R³ is a hydrogen atom or a methyl group.

4. Use of compounds of formula (I) as defined in any of claims 1 to 3, and/or salts thereof, in the manufacture of a medicament for the treatment or prophylaxis of retrovirus infections.

## Patentansprüche (Patentansprüche für folgende(n) Vertragsstaat(en): AT, BE, CH, DE, FR, GB, IT, LI, LU, NL, SE)

1. Verbindungen der Formel (I) 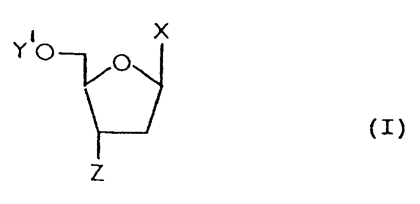 (worin Z ein Wasserstoffatom oder eine Azidogruppe darstellt,
Y¹ ein Wasserstoffatom oder eine physiologisch verträgliche Gruppe der Formel
R¹(O)ₙCO(OCR²R³)ₘ-
darstellt, worin n 0 oder 1 bedeutet, m 0 oder 1 bedeutet und
R¹ eine gegebenenfalls substituierte Alkyl- oder Arylgruppe oder, wenn n 0 ist, ein Wasserstoffatom darstellt.
R² und R³ unabhängig voneinander Wasserstoffatome oder Niederalkylgruppen sind; und
X eine Gruppe ist; ausgewählt aus 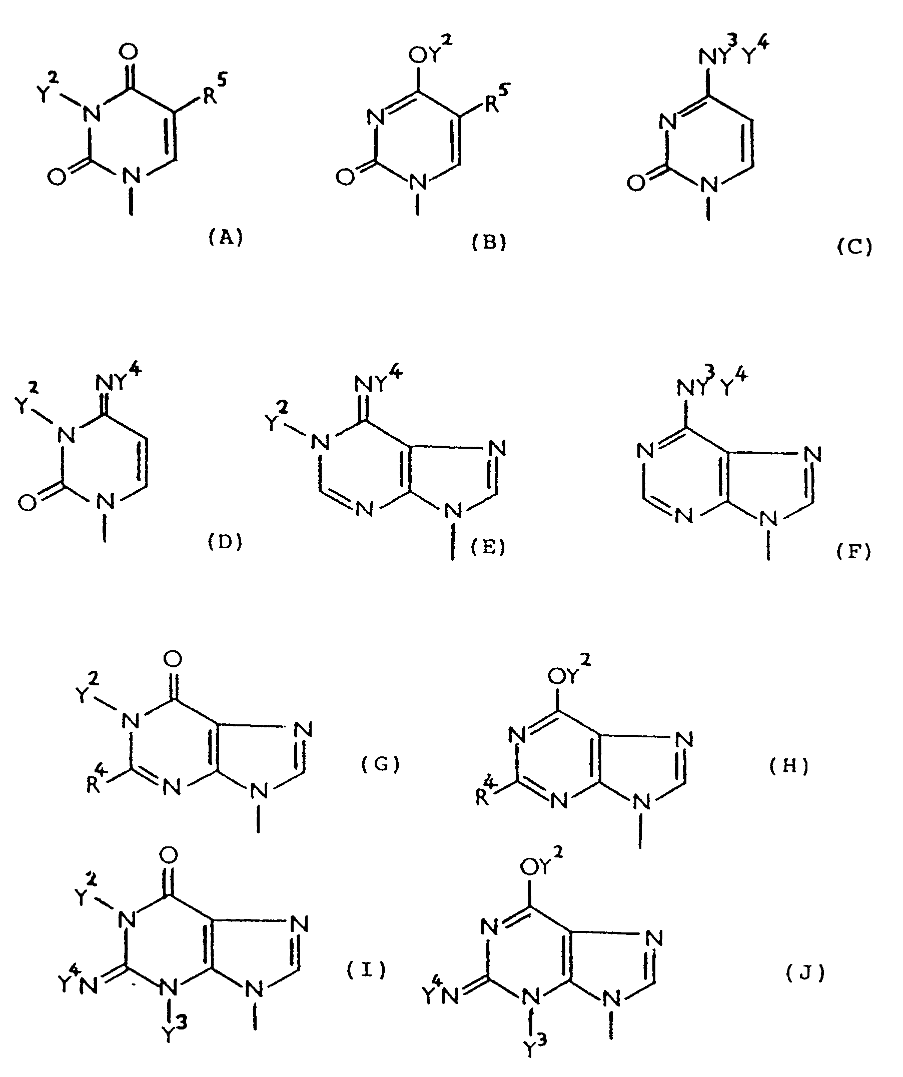 worin die Gruppen Y²_{,} Y³ und Y⁴ wie für Y¹ definiert sind und gleich oder verschieden von Y¹ oder einander sein können, R⁴ ein Wasserstoffatom oder eine Gruppe -NY³Y⁴ ist, wobei Y³ und Y⁴ die vorstehenden Bedeutungen haben und R⁵ ein Wasserstoffatom oder eine Niederalkylgruppe darstellt, mit der Maßgabe, daß mindestens eine der Gruppen Y¹, Y², Y³ und Y⁴ eine Gruppe R¹(O)ₙCO(OCR²R³)ₘ bedeutet, worin m 1 ist) und/oder Salze davon.

2. Verbindungen der Formel (I) nach Anspruch 1, wobei R¹ ausgewählt ist aus gegebenenfalls substituierten C₁₋₂₀-Alkylgruppen und C₆₋₂₀-Arylgruppen.

3. Verbindungen der Formel (I) nach Anspruch 1, wobei R² ein Wasserstoffatom ist; und R³ ein Wasserstoffatom oder eine Methylgruppe bedeutet.

4. Arzneimittel, umfassend als Wirkstoff eine oder mehrere Verbindungen von Formel (I) nach einem vorangehenden Anspruch und/oder ein nichttoxisches Salz davon, zusammen mit einem pharmazeutisch verträglichen Träger oder Exzipienten.

5. Verfahren zur Herstellung einer Verbindung der Formel (I) nach einem der Ansprüche 1 bis 3, umfassend die Umsetzung einer Verbindung der Formel (II) 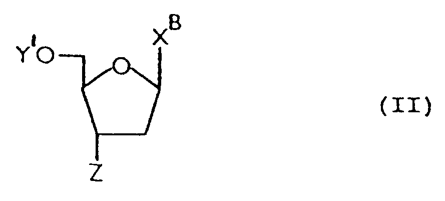 [worin Y¹ und Z wie vorstehend definiert sind und X^{B} wie vorstehend für X definiert ist, mit der Ausnahme, daß jede der Gruppen Y¹, Y², Y³ und Y⁴ zusätzlich eine Schutzgruppe darstellen kann, mit der Maßgabe, daß mindestens ein Rest von Y¹, Y², Y³ und Y⁴ ein Wasserstoffatom ist] mit einem Reagenz, das zur Einführung einer wie vorstehend definierten Gruppe R¹(O)ₙCO(OCR²R³)ₘ, dient, erforderlichenfalls gefolgt von Entfernen von so eingeführten Schutzgruppen und/oder ungewünschten Substituenten.

6. Verwendung von Verbindungen der Formel (I) nach einem der Ansprüche 1 bis 3, und/oder Salzen davon zur Herstellung eines Arzneimittels zur Behandlung oder Prophylaxe von Retrovirusinfektionen.

## Patentansprüche (Patentansprüche für folgende(n) Vertragsstaat(en): ES, GR)

1. Verfahren zur Herstellung von Verbindungen der Formel (I) 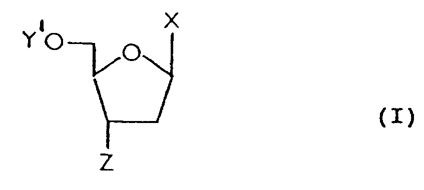 worin Z ein Wasserstoffatom oder eine Azidogruppe darstellt,
Y¹ ein Wasserstoffatom oder eine physiologisch verträgliche Gruppe der Formel
R¹(O)ₙCO(OCR²R³)ₘ-
darstellt, worin n 0 oder 1 bedeutet, m 0 oder 1 bedeutet und
R¹ eine gegebenenfalls substituierte Alkyl- oder Arylgruppe oder, wenn n 0 ist, ein Wasserstoffatom darstellt,
R² und R³ unabhängig voneinander Wasserstoffatome oder Niederalkylgruppen sind; und
X eine Gruppe ist, ausgewählt aus 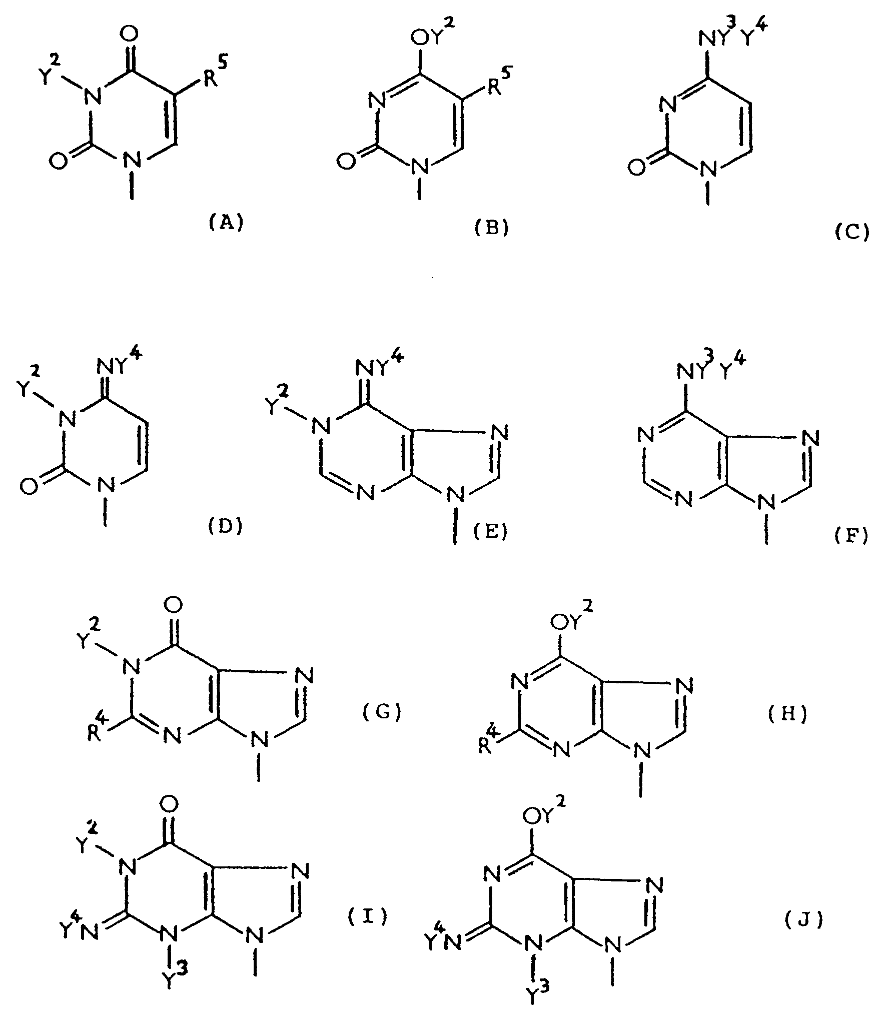 worin die Gruppen Y², Y³ und Y⁴ wie für Y¹ definiert sind und gleich oder verschieden von Y¹ oder einander sein können, R⁴ ein Wasserstoffatom oder eine Gruppe -NY³Y⁴ ist, worin Y³ und Y⁴ die vorstehenden Bedeutungen haben und R⁵ ein Wasserstoffatom oder eine Niederalkylgruppe darstellt, mit der Maßgabe, daß mindestens eine der Gruppen Y¹, Y², Y³ und Y⁴ eine Gruppe R¹(O)ₙCO(OCR²R³)ₘ bedeutet, worin m 1 ist) und/oder Salzen davon, umfassend die Umsetzung einer Verbindung der Formel (II) 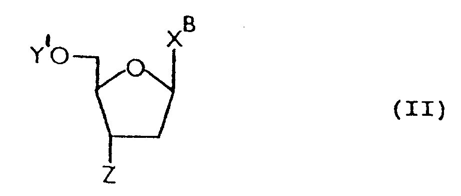 [worin Y¹ und Z wie vorstehend definiert sind und X^{B} wie vorstehend für X definiert ist, mit der Ausnahme, daß jede der Gruppen Y¹, Y²_{,} Y³ und Y⁴ zusätzlich eine Schutzgruppe darstellen kann, mit der Maßgabe, daß mindestens ein Rest von Y¹, Y², Y³ und Y⁴ ein Wasserstoffatom ist] mit einem Reagenz, das zur Einführung einer wie vorstehend definierten Gruppe R¹(O)ₙCO(OCR²R³)ₘ, dient, erforderlichenfalls gefolgt von Entfernen von so eingeführten Schutzgruppen und/oder ungewünschten Substituenten.

2. Verfahren nach Anspruch 1 zur Herstellung von Verbindungen der Formel (I), wobei R¹ ausgewählt ist aus gegebenenfalls substituierten C₁₋₂₀ Alkylgruppen und C₆₋₂₀-Arylgruppen.

3. Verfahren nach Anspruch 1 zur Herstellung von Verbindungen der Formel (I), worin R² ein Wasserstoffatom ist; und R³ ein Wasserstoffatom oder eine Methylgruppe bedeutet.

4. Verwendung von Verbindungen der Formel (I) nach einem der Ansprüche 1 bis 3, und/oder Salzen davon zur Herstellung eines Arzneimittels zur Behandlung oder Prophylaxe von Retrovirusinfektionen.

## Revendications (Revendications pour l'(les) Etat(s) contractant(s) suivant(s): AT, BE, CH, DE, FR, GB, IT, LI, LU, NL, SE)

1. Composés de formule (I) 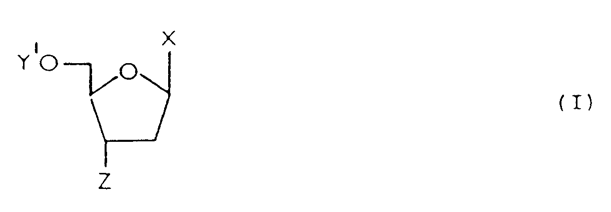 dans laquelle Z est un atome d'hydrogène ou un groupe azido,
Y¹ est un atome d'hydrogène ou un groupe acceptable du point de vue physiologique de formule
R¹(O)ₙCO(OCR²R³)ₘ-
où n vaut O ou 1, m vaut O ou 1 et
R¹ est un groupe alkyle ou aryle éventuellement substitué ou, uand n vaut O, un atome d'hydrogène.
R² et R³ sont indépendamment des atomes d'hydrogène ou des groupes alkyle inférieur ; et
X est un groupe choisi parmi 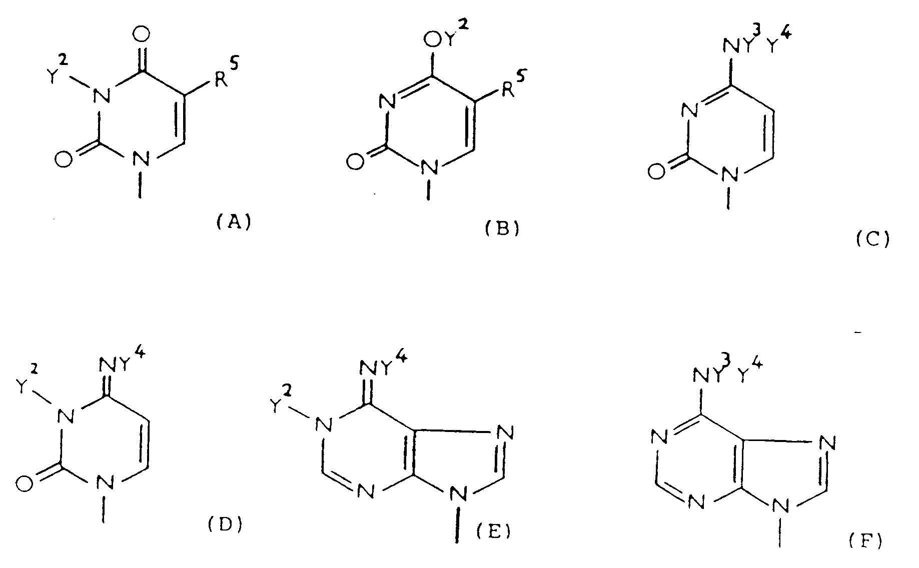 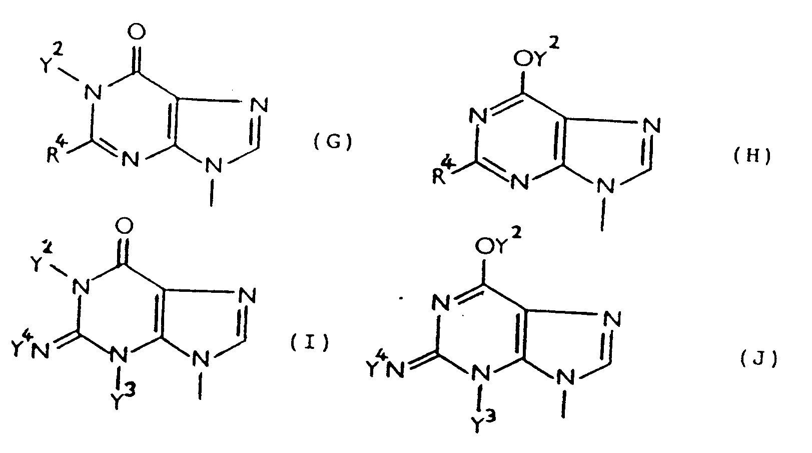 où les groupes Y², Y³ et Y⁴ sont comme définis pour Y¹ et peuvent être identiques ou différents de Y¹ ou les uns des autres, R⁴ est un atome d'hydrogène ou un groupe -NY³Y⁴, où Y³ et Y⁴ ont les significations ci-dessus et R⁵ est un atome d'hydrogène ou un groupe alkyle inférieur. à la condition qu'au moins un des groupes Y¹, Y², Y³ et Y⁴ soit un groupe R¹(O)ₙCO(OCR²R³)ₘ dans lequel m est égal à 1,et/ou les sels de ceux-ci.

2. Composés de formule (I) comme revendiqués dans la revendication 1 dans lesquels R¹ est choisi parmi des groupes alkyle C₁₋₂₀ et des groupes aryle C₆₋₂₀ éventuellement substitués.

3. Composés de formule (I) comme revendiqués dans la revendication 1 dans lesquels R² est un atome d'hydrogène ; et R³ est un atome d'hydrogène ou un groupe méthyle.

4. Composition pharmaceutique comprenant comme ingrédient actif un ou plusieurs composés de formule (I) comme défini dans l'une quelconque des revendications précédentes, et/ou un sel non toxique de ceux-ci. avec un véhicule ou un excipient pharmaceutique.

5. Procédé de préparation d'un composé de formule (I) comme défini dans l'une quelconque des revendications 1 à 3, qui comprend la réaction d'un composé de formule (II) 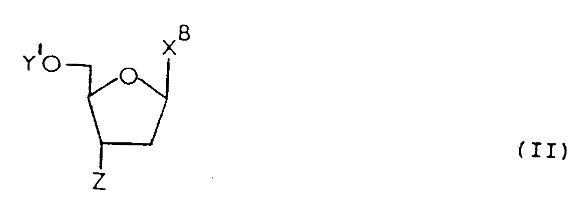 [dans laquelle Y¹ et Z sont comme définis ci-dessus et X^{B} est comme défini ci-dessus pour X à l'exception du fait que l'un quelconque des groupes Y¹, Y², Y³ et Y⁴ peut en outre représenter un groupe protecteur, à la condition qu'au moins un des groupes Y¹, Y²_{,} Y³ et Y⁴ soit un atome d'hydrogène] avec un réactif servant à introduire un groupe R¹(O)ₙCO.(OCR²R³)ₘ comme défini ci-dessus, suivie, si nécessaire, de l'élimination de tous groupes protecteurs et/ou substituants non désirés ainsi introduits.

6. Utilisation de composés de formule (I) code définis dans l'une quelconque des revendications 1 à 3, et/ou des sels de ceux-ci, dans la fabrication d'un médicament pour le traitement ou la prophylaxie d'infections à rétrovirus.

## Revendications (Revendications pour l'(les) Etat(s) contractant(s) suivant(s): ES, GR)

1. Procédé pour la préparation de composés de formule (I) 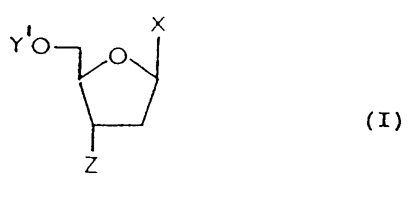 dans laquelle Z est un atome d'hydrogène ou un groupe azido,
Y¹ est un atome d'hydrogène ou un groupe acceptable du point de vue physiologique de formule
R¹(O)ₙCO(OCR²R³)ₘ-
où n vaut O ou 1, m vaut O ou 1 et
R¹ est un groupe alkyle ou aryle éventuellement substitué ou, quand n vaut O, un atome d'hydrogène.
R² et R³ sont indépendamment des atomes d'hydrogène ou des groupes alkyle inférieur ; et
X est un groupe choisi parmi 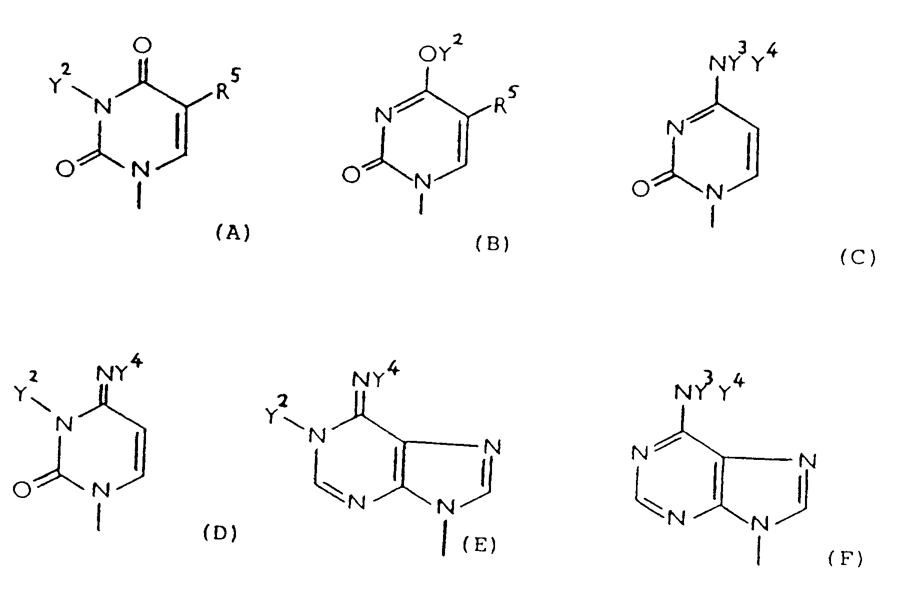 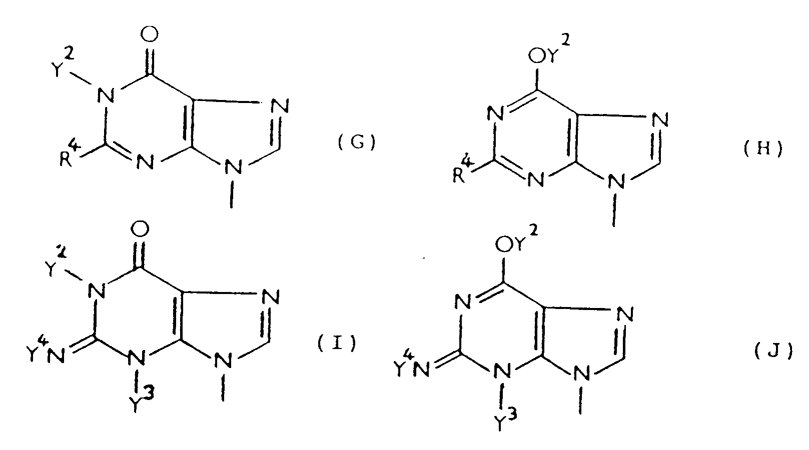 (où les groupes Y², Y³ et Y⁴ sont comme définis pour Y¹ et peuvent être identiques ou différents de Y¹ ou les uns des autres. R⁴ est un atome d'hydrogène ou un groupe -NY³Y⁴. où Y³ et Y⁴ ont les significations ci-dessus et R⁵ est un atome d'hydrogène ou un groupe alkyle inférieur, à la condition qu'au moins un des groupes Y¹, Y², Y³ et Y⁴ soit un groupe R¹(O)ₙCO(OCR²R³)ₘ dans lequel m est égal à 1) et/ou les sels de ceux-ci. comprenant la réaction d'un composé de formule (II) 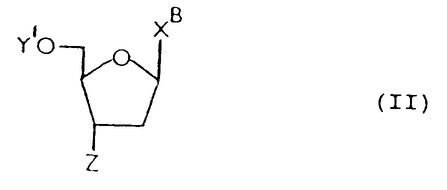 [dans laquelle Y¹ et Z sont comme définis ci-dessus et X^{B} est comme défini ci-dessus pour X à l'exception du fait que l'un quelconque des groupes Y ¹, Y², Y³ et Y⁴ peut en outre représenter un groupe protecteur, à la condition qu'au moins un des groupes Y¹, Y², Y³ et Y⁴ soit un atome d'hydrogène] avec un réactif servant à introduire un groupe R¹(O)ₙCO.(OCR²R³)ₘ comme défini ci-dessus, suivie, si nécessaire, de l'élimination de tous groupes protecteurs et/ou les substituants non désirés ainsi introduits.

2. Procédé comme revendiqué dans la revendication 1 pour la préparation de composés de formule (I) dans lequel R¹ est choisi parmi des groupes alkyle C₁₋₂₀ et des groupes aryle C₆₋₂₀ éventuellement substitués.

3. Procédé comme revendiqué dans la revendication 1 pour la préparation de composés de formule (I) dans lequel R² est un atome d'hydrogène ; et R³ est un atome d'hydrogène ou un groupe méthyle.

4. Utilisation de composés de formule (I) comme définis dans l'une quelconque des revendications 1 à 3, et/ou de sels de ceux-ci, dans la fabrication d'un médicament pour le traitement ou la prophylaxie d'infections à rétrovirus.
